# EUROPÄISCHE PATENTSCHRIFT

(11) **EP 1 055 655 B1**
(45) Veröffentlichungstag und Bekanntmachung des Hinweises auf die Patenterteilung: **14.06.2006**
(21) Anmeldenummer: 00110914.9
(22) Anmeldetag: 24.05.2000
(51) Int. Cl.: C07C 29/42, C07C 33/044

(54) **Verfahren zur Herstellung von Alkindiolen**
Process for the preparation of alkynediols
Procédé de préparation d'alkynediols

(30) Priorität: 26.05.1999 DE 19924020
(43) Veröffentlichungstag der Anmeldung: 29.11.2000
(73) Patentinhaber: BASF Aktiengesellschaft, 67056 Ludwigshafen (DE)
(72) Erfinder: Kindler, Alois, Dr., 57269 Grünstadt (DE); Preiss, Thomas, Dr., 67256 Weisenheim am Sand (DE); Henkelmann. Jochem, Dr., 68165 Mannheim (DE)
(74) Vertreter: Isenbruck, Günter

(56) Entgegenhaltungen:
- EP-A- 0 285 755
- WO-A-00/09465
- DE-A- 19 635 703
- TEDESCHI, R.J.; ET AL.: J. ORG. CHEM., Bd. 28, 1963, Seiten 1740-1743, XP002259149
- BABLER, J.H.; ET AL.: J. ORG. CHEM., Bd. 61, 1996, Seiten 416-471, XP002259150
- TZALIS D: "Cesium hydroxide: a superior base for the catalytic alkynylation of aldehydes and ketones and catalytic alkenylation of nitriles" ANGEWANDTE CHEMIE. INTERNATIONAL EDITION, VERLAG CHEMIE. WEINHEIM, DE, Bd. 38, Nr. 10, 17. Mai 1999 (1999-05-17), Seiten 1463-1465, XP002147490 ISSN: 0570-0833
- DATABASE CA [Online] CHEMICAL ABSTRACTS SERVICE, COLUMBUS, OHIO, US; WEN, ZHANG ET AL: "Nonsupported catalysts for producing 1,4-butynediol and their preparation and application" retrieved from STN Database accession no. 130:254049 XP002259151 & CN 1 118 342 A (BEIJING CHEMICAL INDUSTRY INSTITUTE, MINISTRY OF CHEMICAL INDUSTRY, PE) 13. März 1996 (1996-03-13)

## Beschreibung

Die Erfindung betrifft ein Verfahren zur Herstellung von Alkindiolen durch Umsetzung von Acetylen mit Carbonylverbindungen in Gegenwart basischer Katalysatoren.

Alkindiole sind wertvolle Zwischenprodukte zur Herstellung von beispielsweise schaumarmen Tensiden, Pyrethroiden, Galvanohilfsmitteln oder Peroxiden.

Zur Herstellung von Alkinolen sind unterschiedliche Herstellungsverfahren bekannt. Sie können grob in Übergangsmetall-katalysierte und Basen-katalysierte Umsetzungen unterteilt werden.

In W. Reppe, Liebigs Anm. Chem. 1955, 596, Seiten 1 bis 3, wird beispielsweise die Carbonylierung von Acetylen mit Carbonyl-Verbindungen in Gegenwart von Acetyliden der Schwermetalle der ersten Nebengruppe des Periodensystems der Elemente in THF beschrieben.

Auf den Seiten 6 bis 11 und 25 bis 38 wird beschrieben, daß die Ethinylierung von Ketonen in wäßrigen Medien unter Verwendung von Alkalihydroxiden, Erdalkalihydroxiden, Alkalicarbonaten oder tertiären Aminen als Katalysatoren durchgeführt werden kann. Zudem kann Kupferacetylid als Katalysator verwendet werden. In den Beispielen wird die Herstellung von Methylbutinol aus Aceton und Acetylen in Wasser mit Kaliumhydroxid als Katalysator erwähnt.

R.J. Tedeschi et al., J. Org. Chem. 1963, 28 Seiten 1715 bis 1743 beschrieben die Basen-katalysierte Umsetzung von Acetylen und Phenylacetylenen mit Carbonyl-Verbindungen in flüssigem Ammoniak unter Druck. Dabei wird die Umsetzung zu den entsprechenden sekundären und tertiären acetylenischen Carbinolen unter Verwendung katalytischer Mengen von Natrium- oder Kaliumhydroxid durchgeführt.

In B. A. Trofimov, Russian Journal of Organic Chemistry, Vol. 31, No. 9, 1995, Seiten 1233 bis 1252 sind unterschiedliche Umsetzungen mit Acetylen beschrieben. Unter anderem sind Ethinylierungen unter Verwendung des superbasischen Systems KOH/DMSO aufgeführt.

J. H. Babler et al., J. Org. Chem. 1996, 61 Seiten 416 bis 417 beschreiben die Alkoxid-katalysierte Addition von terminalen Alkinen an Ketone. Dabei wird die Umsetzung mit tert.-Butoxid als Katalysator durchgeführt. Die Herstellung von Alkindiolen ist nicht erwähnt.

Alkindiole wurden bislang nur unter Verwendung mindestens stöchiometrischer Mengen an Base hergestellt. Hierzu werden häufig nucleophile Kaliumbasen in aprotischen Lösungsmitteln eingesetzt, siehe J. Org. Chem. 1963, 28, Seiten 2480 bis 2483 und J. Appl. Chem. 1953, Seiten 39 bis 42, wie auch EP-A-0 285 755.

DE 196 35 703 A1 offenbart Alkindiole oder Gemische von Alkindiolen mit Alkinmonoolen, welche durch Umsetzung von Acetylen mit mehr als äquimolaren Mengen an Ketonen und/oder Aldehyden in Gegenwart einer Alkaliverbindung hergestellt werden.

D. Tzalis und P. Knochel offenbaren in Angew. Chem. Int. Ed. 1999, 38, No. 10, die Verwendung von Caesiumhydroxid als geeignete Base bei der katalytischen Alkinylierung von Aldehyden und Ketonen. Caesiumhydroxid wird in einer Menge von 10 - 30 mol% eingesetzt.

Database CA Online, XP 002259151, Wen, Zhang et al. offenbart die Verwendung einer Mischung aus Kupferoxid und Bismutoxid als Katalysator bei der Reaktion von Formaldehyd mit Acetylen.

EP 0 285 755 A2 offenbart ein Verfahren zur Herstellung von Alkindiolen durch Umsetzung von Ketonen und Aldehyden in Gegenwart einer Base. Die Verwendung von Alkyl-tert.-butylethern als Lösungsmittel wird offenbart.

Aufgabe der vorliegenden Erfindung ist die Bereitstellung eines Verfahrens zur Herstellung von Alkindiolen, das die Nachteile der bestehenden Verfahren vermeidet und nur das Vorliegen katalytischer Mengen an Basen erfordert.

Die Aufgabe wird erfindungsgemäß gelöst durch ein Verfahren zur Herstellung von Alkindiolen der allgemeinen Formel (I)

R¹R²C(OH)-C≡C-C(OH)R¹R² (I)

mit der Bedeutung
- R¹, R²: unabhängig H oder C₁₋₂₀-Kohlenwasserstoffrest, der ein- oder mehrfach durch C₁₋₆-Alkyl substituiert und/oder von nicht benachbarten Heteroatomen unterbrochen sein und/oder C-C-Doppel- oder Dreifachbindungen enthalten kann,
durch Umsetzung von Verbindungen der allgemeinen Formel (II)

R¹-C(=O)-R² (II)

mit Acetylen in einem polar aprotischen Lösungsmittel in Gegenwart von Alkalimetallalkoholaten, in denen die zugrundeliegenden Alkohole ausgewählt sind aus Alkinolen der allgemeinen Formel (III)

R¹R²C(OH)-C≡CH (III)

mit der vorstehend angegebenen Bedeutung für R¹ und R² als basischen Katalysatoren.

Es wurde gefunden, daß unter Verwendung von Alkalimetallalkoholaten, in denen die zugrundeliegenden Alkohole ausgewählt sind aus Alkinolen der allgemeinen Formel (III)

R¹R²C(OH)-C≡CH (III)

mit der vorstehend angegebenen Bedeutung für R¹ und R² in polar aprotischen Lösungsmitteln Alkindiole in hoher Ausbeute zugänglich sind.

Bei den Alkalimetallalkoholaten kann es sich um Lithium-, Natrium-, Kalium-, Rubidium oder Cäsiumalkoholate handeln. Vorzugsweise werden Kaliumalkoholate als basische Katalysatoren eingesetzt.

Der basische Katalysator kann auch in situ aus Alkalimetallhydriden, Acetylen und der Verbindung der allgemeinen Formel (II) hergestellt werden.

Bei der Umsetzung werden neben Alkindiolen in gewissen Mengen auch Alkinole der allgemeinen Formel (III) gebildet. Deshalb kann sich der Einsatz dieser Verbindungen oder deren in situ-Herstellung anbieten.

Der basische Katalysator wird vorzugsweise in einer Menge von 5 bis 65 mol-%, besonders bevorzugt 10 bis 30 mol-%, bezogen auf Acetylen, eingesetzt.

Die Umsetzung erfolgt vorzugsweise bei einer Temperatur im Bereich von -20 bis +60°C, besonders bevorzugt -10 bis + 40°C, insbesondere 10 bis 30°C und einem Druck von 0,1 bis 10 bar, besonders bevorzugt 0,5 bis 5 bar, insbesondere Normaldruck.

Das polar aprotische Lösungsmittel ist vorzugsweise ausgewählt aus Tetrahydrofuran (THF), N-Methylpyrrolidon (NMP), Dimethylsulphoxid (DMSO) und Gemischen davon, wobei bis zu 20 Gew.-% des Lösungsmittels durch unpolare Kohlenwasserstoffe ersetzt sein können. Besonders bevorzugt basiert das Lösungsmittel auf THF. Insbesondere liegt der Anteil unpolarer Kohlenwasserstoffe unter 10 Gew.-%, speziell unter 5 Gew.-%. Insbesondere wird nur THF als Lösungsmittel eingesetzt.

Für die erfindungsgemäße Umsetzung können unterschiedlichste Carbonylverbindungen der allgemeinen Formel (II) eingesetzt werden. Vorzugsweise haben R¹ und R² unabhängig die Bedeutung H oder C₁₋₁₂-Kohlenwasserstoffrest, der geradkettig oder verzweigt sein kann. Besonders bevorzugt bedeuten R¹ und R² unabhängig H oder C₁₋₆-Alkyl. Beispiele geeigneter Carbonylverbindungen sind Formaldehyd und Aceton.

Im erfindungsgemäßen Verfahren kann vorzugsweise der basische Katalysator im polaren aprotischen Lösungsmittel vorgelegt werden, worauf Acetylen und die Carbonylverbindungen parallel zudosiert und umgesetzt werden. Sodann kann hydrolytisch aufgearbeitet werden.

Die Erfindung wird nachstehend anhand von Beispielen näher erläutert.

### BEISPIELE:

### Beispiel 1 Herstellung von Butindiol

In einen mit Rührer versehenen Doppelmantelreaktor wurden 400 g THF und 4 g Kaliumhydrid sowie 5,6 g Propinol bei 30°C eingefüllt und 15 min bei dieser Temperatur gerührt. Die Lösung wurde auf 10°C eingestellt und während der Reaktion bei dieser Temperatur gehalten. Parallel wurde über 1 Stunde 60 g paraFormaldehyd (2 mol) und 26 g Acetylen (1 mol) eingeleitet. Nach 15 min Nachreaktion wurde mit 150 g Wasser hydrolysiert. Nach der Phasentrennung wurde die wässrige Phase mit H₃PO₄ angesäuert, dreimal mit Diethylether extrahiert, und die Extrakte wurden mit der organischen Phase vereinigt und analysiert. Es wurden 3,83 Gew.-%, bezogen auf Formaldehyd, an Rückstand gefunden. Der Umsatz an Formaldehyd betrug 96%. Es wurde eine Ausbeute von 76% Propinol und 20% Butindiol gefunden.

### Beispiel 2 Herstellung von Dimethylhexindiol

In einen mit Rührer versehenen Doppelmantelreaktor wurden 400 g THF und 12 g Kaliumhydrid (0,3 mol) sowie 34 g iso-Butanol (0,3 mol) bei 30°C eingefüllt und 15 min bei dieser Temperatur gerührt. Die Lösung wurde bei 30°C temperiert und während der Reaktion bei dieser Temperatur gehalten. Parallel wurden über 2 Stunden 58 g Aceton (1 mol) und 26 g Acetylen (1 mol) eingeleitet. Nach 15 min Nachreaktion wurde mit 150 g Wasser hydrolysiert. Nach der Phasentrennung wurde die wässrige Phase mit H₃PO₄ angesäuert, dreimal mit Diethylether extrahiert, und die Extrakte wurden mit der organischen Phase vereinigt und analysiert. Der Umsatz an Aceton betrug 96%. Es wurde eine Ausbeute von 74% Methylbutinol und 22% Dimethylhexindiol gefunden.

### Beispiel 3 Herstellung von Dimethylhexindiol

In einen mit Rührer versehenen Doppelmantelreaktor wurden 400 g THF und 120 g Kalium-isobutylat in Xylol (0,6 mol) eingefüllt. Die Suspension wurde bei 30°C temperiert und während der Reaktion bei dieser Temperatur gehalten. Parallel wurden über 2 Stunden 116 g Aceton (2 mol) und 26 g Acetylen (1 mol) eingeleitet. Nach 15 min Nachreaktion wurde mit 150 g Wasser hydrolysiert. Nach der Phasentrennung wurde die wässrige Phase verworfen. Die organische Phase wurde analysiert. Der Umsatz an Aceton betrug 75%. Es wurde eine Ausbeute von 19% Methylbutinol und 56% Dimethylhexindiol gefunden.

### Beispiel 4 Herstellung von Dimethylhexindiol

In einen mit Rührer versehenen Doppelmantelreaktor wurden 400 g THF und 24 g Kaliumhydrid (0,6 mol) eingefüllt. Die Suspension wurde bei 30°C temperiert und während der Reaktion bei dieser Temperatur gehalten. Parallel wurden über 2 Stunden 116 g Aceton (2 mol) und 26 g Acetylen (1 mol) eingeleitet. Nach 15 min Nachreaktion wurde mit 150 g Wasser hydrolysiert. Nach der Phasentrennung wurde die wässrige Phase verworfen. Die organische Phase wurde analysiert. Der Umsatz an Aceton betrug 97%. Es wurde eine Ausbeute von 7% Methylbutinol und 91% Dimethylhexindiol gefunden.

Die Umsetzungen wurden jeweils bei einem Druck von 1013 mbar durchgeführt.

## Patentansprüche

1. Verfahren zur Herstellung von Alkindiolen der allgemeinen Formel (I)
R¹R²C(OH)-C≡C-C(OH)R¹R² (I)
mit der Bedeutung
R¹, R² unabhängig H oder C₁₋₂₀-Kohlenwasserstoffrest, der ein- oder mehrfach durch C₁₋₆-Alkyl substituiert und/oder von nicht benachbarten Heteroatomen unterbrochen sein und/oder C-C-Doppel- oder Dreifachbindungen enthalten kann,
durch Umsetzung von Verbindungen der allgemeinen Formel (II)
R¹-C(=O)-R² (II)
mit Acetylen in einem polar aprotischen Lösungsmittel in Gegenwart von Alkalimetallalkoholaten, in denen die zugrundeliegenden Alkohole ausgewählt sind aus Alkinolen der allgemeinen Formel (III)
R¹R²C(OH)-C≡CH (III)
mit der angegebenen Bedeutung für R¹ und R², als Katalysatoren.

2. Verfahren nach Anspruch 1, **dadurch gekennzeichnet, daß** Kaliumalkoholate als basische Katalysatoren eingesetzt werden.

3. Verfahren nach Anspruch 1, **dadurch gekennzeichnet, daß** der basische Katalysator in situ aus Alkalimetallhydriden, Acetylen und der Verbindung der allgemeinen Formel (II) hergestellt wird.

4. Verfahren nach einem der Ansprüche 1 bis 3, **dadurch gekennzeichnet, daß** der basische Katalysator in einer Menge von 5 bis 65 mol-%, bezogen auf Acetylen, eingesetzt wird.

5. Verfahren nach einem der Ansprüche 1 bis 4, **dadurch gekennzeichnet, daß** die Umsetzung bei einer Temperatur in einem Bereich von -20 bis 60°C und einem Druck im Bereich von 0,1 bis 10 bar durchgeführt wird.

6. Verfahren nach einem der Ansprüche 1 bis 5, **dadurch gekennzeichnet, daß** das polar aprotische Lösungsmittel ausgewählt ist aus THF, NMP, DMSO und Gemischen davon, wobei bis zu 20 Gew.-% des Lösungsmittels durch unpolare Kohlenwasserstoffe ersetzt werden können.

7. Verfahren nach einem der Ansprüche 1 bis 6, **dadurch gekennzeichnet, daß** R¹und R² unabhängig H oder C₁₋₆-Alkyl bedeuten.

## Claims

1. A process for preparing alkynediols of the formula (I)
R¹R²C(OH)-C≡C-C(OH)R¹R² (I)
where
R¹, R² are each independently H, or a C₁₋₂₀₋hydrocarbon radical which may be substituted by one or more C₁₋₆-alkyls and/or be interrupted by nonadjacent heteroatoms and/or comprise C-C double or triple bonds,
by reacting compounds of the formula (II)
R¹-C(=O)-R² (II)
with acetylene in a polar aprotic solvent in the presence of alkali metal alkoxides in which the parent alcohols are selected from among alkynols of the formula (III)
R¹R²C(OH)-C≡CH (III)
where R1 and R2 are as defined above, as catalyst.

2. The process according to claim 1, wherein potassium alkoxides are used as basic catalysts.

3. The process according to claim 1, wherein the basic catalyst is prepared in situ from alkali metal hydrides, acetylene and the compound of the formula (II).

4. The process according to any of claims 1 to 3, wherein the basic catalyst is used in an amount of from 5 to 65 mol%, based on acetylene.

5. The process according to any of claims 1 to 4, wherein the reaction is carried out at from -20 to 60°C and at a pressure in the range from 0.1 to 10 bar.

6. The process according to any of claims 1 to 5, wherein the polar aprotic solvent is selected from among THF, NMP, DMSO and mixtures thereof, with up to 20% by weight of the solvent being able to be replaced by nonpolar hydrocarbons.

7. The process according to any of claims 1 to 6, wherein R¹ and R² are independently H or C₁₋₆-alkyl.

## Revendications

1. Procédé de préparation d'alcynediols de la formule générale (I) :
R¹R²C(OH)-C≡C-C(OH)R¹R² (I)
dans laquelle
R¹, R² représentent indépendamment H ou un radical d'hydrocarbure en C₁-C₂₀, qui peut être substitué à une ou à plusieurs reprises par un groupe alkyle en C₁-C₆ et/ou être interrompu par des hétéroatomes non voisins et/ou contenir des doubles ou triples liaisons C-C,
par réaction de composés de la formule générale (II) :
R¹-C(=O)-R² (II)
avec de l'acétylène dans un solvant aprotique polaire, en présence d'alcoolates de métal alcalin, dans lesquels les alcools constitutifs sont choisis parmi des alcynols de la formule générale (III) :
R¹R²C(OH)-C≡CH (III)
dans laquelle R¹ et R² ont la signification indiquée, comme catalyseurs.

2. Procédé suivant la revendication 1, **caractérisé en ce que**, comme catalyseurs basiques, on met en oeuvre des alcoolates de potassium.

3. Procédé suivant la revendication 1, **caractérisé en ce que** le catalyseur basique est préparé in situ à partir d'hydrures de métal alcalin, d'acétylène et du composé de la formule générale (II).

4. Procédé suivant l'une des revendications 1 à 3, **caractérisé en ce que** le catalyseur basique est mis en oeuvre en une quantité de 5 à 65 % molaires, par rapport à l'acétylène.

5. Procédé suivant l'une des revendications 1 à 4, **caractérisé en ce que** la réaction est effectuée à une température de l'ordre de -20 à 60°C et à une pression de l'ordre de 0,1 à 10 bars.

6. Procédé suivant l'une des revendications 1 à 5, **caractérisé en ce que** le solvant aprotique polaire est choisi parmi du THF, du NMP, du DMSO et leurs mélanges, jusqu'à 20 % en poids du solvant pouvant être remplacés par des hydrocarbures non polaires.

7. Procédé suivant l'une des revendications 1 à 6, **caractérisé en ce que** R¹ et R² représentent indépendamment H ou un groupe alkyle en C₁-C₆.
